# EUROPEAN PATENT APPLICATION

(11) **EP 3 581 933 A2**
(43) Date of publication of application: **18.12.2019**
(21) Application number: 19174974.6
(22) Date of filing: 16.05.2019
(51) Int. Cl.: G01N 33/574, G01N 33/68

(54) **IDENTIFICATION OF CANCER PATIENTS SENSITIVE OR RESISTANT TO 5-FLUOROURACIL (5-FU)**

(30) Priority: 17.05.2018 US 201862672671 P
(71) Applicant: NantOmics, LLC, Culver City, CA 90232 (US)
(72) Inventor: HEMBROUGH, Todd A., Culver City, CA California 90232 (US); CECCHI, Fabiola, Culver City, CA California 90232 (US); SCOTT, Kerry Mina, Culver City, CA California 90232 (US)
(74) Representative: AWA Sweden AB

(57) **Abstract**

Methods are provided for quantifying the TYMS, TYMP, UCK1, UCK2, UPP1, OPRT, dCK, TK1, DPYD, CDA, RRM1, and/or RRM2 proteins directly in biological samples that have been fixed in formalin by the method of Selected Reaction Monitoring/ Multiple Reaction Monitoring (SRM/MRM). The biological samples are chemically preserved with formaldehyde-containing agents/fixatives including formalin-fixed tissue/cells, formalin-fixed/paraffin embedded (FFPE) tissue/cells, FFPE tissue blocks and cells from those blocks, and tissue culture cells that have been formalin fixed and or paraffin embedded. A protein digest sample is prepared from the biological sample and the TYMS, TYMP, UCK1, UCK2, UPP1, OPRT, dCK, TK1, DPYD, CDA, RRM1, and/or RRM2 proteins are quantitated in the digest by SRM/MRM mass spectrometry.

## Description

### FIELD

Methods are provided for measuring expression of multiple proteins having functions that can affect or determine a cancer patient's response to treatment with 5-fluorouracil (5-FU). The proteins are TYMS, TYMP, UCK1, UCK2, UPP1, OPRT, dCK, TK1, DPYD, CDA, RRM1, and RRM2.

### DETAILED DESCRPTION

The level of protein expression of one or more of the proteins TYMS, TYMP, UCK1, UCK2, UPP1, OPRT, dCK, TK1, DPYD, CDA, RRM1, and RRM2 in patient tumor tissue is determined by quantitating a specified peptide derived from subsequences of each of the full-length proteins. Each peptide is detected using mass spectrometry-based Selected Reaction Monitoring (SRM), also referred to as Multiple Reaction Monitoring (MRM), and which is referred to herein as an SRM/MRM assay. An SRM/MRM assay is used to detect the presence and quantitatively measure the amount of a specified fragment peptide, directly in cells procured from cancer patient tissue, such as, for example formalin fixed cancer tissue.

The quantitation is relative or absolute. When absolute quantitation is required the measured level of each peptide is compared to a known amount of a labeled reference peptide having the same amino acid sequence as the measured peptide. The peptides are unique to a specific protein and therefore one peptide molecule is derived from one protein molecule and therefore the quantitative level of the peptide allows quantitation of the intact protein from which the peptide is derived. The measurements of protein expression can be used for diagnosis of cancer, staging of the cancer, prognosis of cancer progression, predicting the likelihood of clinical response to various cancer treatments and therapies, and the like.

### Measured proteins

TYMS (also known as thymidylate synthetase [TS]) is an enzyme that catalyzes the conversion of deoxyuridine monophosphate (dUMP) to deoxythymidine monophosphate (dTMP). With inhibition of TYMS due to treatment of the cell with 5-FU, an imbalance of deoxynucleotides and increased levels of dUMP arise causing DNA damage and the inability to synthesize DNA. TYMS is a target of cancer chemotherapy and overexpression of TYMS in cancer causes resistance to DNA damaging agents such as 5-FU. Thus the presence and quantitative levels of TYMS will indicate if the tumor cells may be sensitive or resistance to treatment with 5-FU. Accordingly, an assay that can quantitate the TYMS protein in tumor cells is useful as part of a cancer therapy regimen and, in particular, information about the level of TYMS expression can be used in selecting specific methods of treatment.

TYMP (also known as thymidine phosphorylase [TP], platelet-derived endothelial cell growth factor [ECGF1], and gliostatin) is an enzyme that catalyzes the reversible phosphorylation of thymidine, deoxyuridine, and their analogs (except deoxycytidine) to their respective bases (thymine/uracil) and 2-deoxyribose 1-phosphate and plays a key role in pyrimidine salvage to recover nucleosides after DNA/RNA degradation. TYMP has been shown to play an essential role in the activation of 5-FU to promote TYMS inhibition. High expression of TYMP in patient tumor cells would indicate that those tumor cells may likely be sensitive to treatment with 5-FU, while low levels may likely promote resistance to 5-FU. Thus the presence and quantitative levels of TYMP will indicate if the tumor cells may be sensitive or resistance to treatment with 5-FU. Accordingly, an assay that can quantitate the TYMP protein in tumor cells is useful as part of a cancer therapy regimen and, in particular, information about the level of TYMP expression can be used in selecting specific methods of treatment.

UCK1 (also known as uridine-cytidine kinase 1, URK1, Uridine monophosphokinase 1) is an enzyme that converts uridine and cytidine to their corresponding monophosphates and is a rate-limiting enzyme involved in the salvage pathway of pyrimidine-nucleotide biosynthesis. UCK1 has been shown to be an integral part of the fluoropyrimidine pathway and when highly expressed in a tumor cell promotes greater sensitivity to treatment with 5-FU whereas lower to no expression helps confer resistance of tumor cells to treatment with 5-FU. Thus the presence and quantitative levels of UCK1 will indicate if the tumor cells may be sensitive or resistance to treatment with 5-FU. Accordingly, an assay that can quantitate the UCK1 protein in tumor cells is useful as part of a cancer therapy regimen and, in particular, information about the level of UCK1 expression can be used in selecting specific methods of treatment.

UCK2 (also known as uridine-cytidine kinase 2) is an enzyme that catalyzes the phosphorylation of uridine and cytidine to uridine monophosphate (UMP) and cytidine monophosphate (CMP), respectively. This is the first step in the production of the pyrimidine nucleoside triphosphates required for RNA and DNA synthesis. UCK2 has been shown to be an integral part of the fluoropyrimidine pathway and when highly expressed in a tumor cell promotes greater sensitivity to treatment with 5-FU whereas lower to no expression helps confer resistance of tumor cells to treatment with 5-FU. Thus the presence and quantitative levels of UCK2 will indicate if the tumor cells may be sensitive or resistance to treatment with 5-FU. Accordingly, an assay that can quantitate the UCK2 protein in tumor cells is useful as part of a cancer therapy regimen and, in particular, information about the level of UCK2 expression can be used in selecting specific methods of treatment.

UPP1 (also known as uridine phosphorylase 1) is an enzyme in the fluoropyrimidine pathway that catalyzes the reversible phosphorylytic cleavage of uridine and deoxyuridine to uracil and ribose- or deoxyribose-1-phosphate. The produced molecules are then utilized as carbon and energy sources or in the rescue of pyrimidine bases for nucleotide synthesis. When highly expressed in a tumor cell UPP1 promotes resistance of tumor cells to treatment with 5-FU whereas lower to no expression helps confer sensitivity of the tumor cells to treatment with 5-FU. Thus the presence and quantitative levels of UPP1 will indicate if the tumor cells may be sensitive or resistance to treatment with 5-FU. Accordingly, an assay that can quantitate the UPP1 protein in tumor cells is useful as part of a cancer therapy regimen and, in particular, information about the level of UPP1 expression can be used in selecting specific methods of treatment.

OPRT (also known as orotate phosphoribosyltransferase, UMP synthase, OMPdecase) is an enzyme in the fluoropyrimidine pathway that catalyzes the formation of uridine monophosphate (UMP), an energy-carrying molecule in many important biosynthetic pathways including the fluoropyrimidine pathway. When highly expressed in a tumor cell OPRT promotes greater sensitivity to treatment with 5-FU whereas lower to no expression helps confer resistance in tumor cells to treatment with 5-FU. Thus the presence and quantitative levels of OPRT will indicate if the tumor cells may be sensitive or resistance to treatment with 5-FU. Accordingly, an assay that can quantitate the OPRT protein in tumor cells is useful as part of a cancer therapy regimen and, in particular, information about the level of OPRT expression can be used in selecting specific methods of treatment.

dCK (also known as deoxycytidine kinase) is an enzyme in the fluoropyrimidine pathway that predominantly phosphorylates deoxycytidine (dC) and converts dC into deoxycytidine monophosphate. dCK catalyzes one of the initial steps in the nucleoside salvage pathway and has the potential to phosphorylate other preformed nucleosides, specifically deoxyadenosine (dA) and deoxyguanosine (dG), and convert them into their monophosphate forms. When highly expressed in a tumor cell dCK promotes greater sensitivity to treatment with 5-FU whereas lower to no expression helps confer resistance of tumor cells to treatment with 5-FU. Accordingly, an assay that can quantitate the dCK protein in tumor cells is useful as part of a cancer therapy regimen and, in particular, information about the level of dCK expression can be used in selecting specific methods of treatment.

TK1 (also known as tyrosine kinase 1) is an enzyme in the fluoropyrimidine pathway that catalyzes the reaction: FUDR + ATP → FdUMP + ADP, where FUDR is 5-fluorodeoxyuridine, ATP is adenosine triphosphate, FdUMP is fluorodeoxyuridine monophosphate and ADP is adenosine diphosphate. TK1 has a key function in the synthesis of DNA and thereby in cell division, as it functions as part of the unique reaction chain to introduce thymidine into the DNA. In the fluoropyrimidine pathway it converts FUDR to FdUMP which directly inhibits TYMS leading to tumor cell death. Thus, when highly expressed in a tumor cell TK1 promotes higher sensitivity to treatment with 5-FU whereas lower to no expression helps confer resistance of tumor cells to treatment with 5-FU. Thus the presence and quantitative levels of TK1 will indicate if the tumor cells may be sensitive or resistance to treatment with 5-FU. Accordingly, an assay that can quantitate the TK1 protein in tumor cells is useful as part of a cancer therapy regimen and, in particular, information about the level of TK1 expression can be used in selecting specific methods of treatment.

DPYD (also known as dihydropyrimidine dehydrogenase, DPD) is a pyrimidine catabolic enzyme and the initial and rate-limiting factor in the pathway of uracil and thymidine catabolism. Deficiency of this enzyme results in an error in pyrimidine metabolism associated with thymine-uraciluria and an increased risk of toxicity in cancer patients receiving 5-fluorouracil chemotherapy. When highly expressed in a tumor cell DPYD helps promote resistance of tumor cells to treatment with 5-FU whereas lower to no expression helps confer sensitivity of the tumor cells to treatment with 5-FU. Thus the presence and quantitative levels of DPYD will indicate if the tumor cells may be sensitive or resistance to treatment with 5-FU. Accordingly, an assay that can quantitate the DPYD protein in tumor cells is useful as part of a cancer therapy regimen and, in particular, information about the level of DPYD expression can be used in selecting specific methods of treatment.

CDA (also known as cytidine deaminase, CDD) is an enzyme involved in pyrimidine salvaging. The encoded protein forms a homotetramer that catalyzes the irreversible hydrolytic deamination of cytidine and deoxycytidine to uridine and deoxyuridine, respectively. It is one of several deaminases responsible for maintaining the cellular pyrimidine pool. In the fluoropyrimidine pathway, CDA catalyzes the reaction 5-dFCR → 5-dFUR which promotes the processing of -5FU in the cell. Thus high expression levels of CDA in a tumor cell promotes higher sensitivity to treatment with 5-FU whereas lower to no expression helps confer resistance of tumor cells to treatment with 5-FU. Thus the presence and quantitative levels of CDA will indicate if the tumor cells may be sensitive or resistance to treatment with 5-FU. Accordingly, an assay that can quantitate the CDA protein in tumor cells is useful as part of a cancer therapy regimen and, in particular, information about the level of CDA expression can be used in selecting specific methods of treatment.

RRM1 (also known as ribonucleotide reductase catalytic subunit M1) is one of two non-identical subunits (with RRM2) which constitute ribonucleoside-diphosphate reductase, an enzyme essential for the production of deoxyribonucleotides prior to DNA synthesis in S phase of dividing cells. In the fluoropyrimidine pathway, CDA catalyzes the reaction FUDP → FdUDP which promotes the processing of 5-FU in the cell to ultimately inhibit DNA synthesis. Thus high expression levels of ribonucleoside-diphosphate reductase in a tumor cell promotes higher sensitivity to treatment with 5-FU whereas lower to no expression helps confer resistance of tumor cells to treatment with 5-FU. Thus the presence and quantitative levels of RRM1 will indicate if the tumor cells may be sensitive or resistance to treatment with 5-FU. Accordingly, an assay that can quantitate the RRM1 protein in tumor cells is useful as part of a cancer therapy regimen and, in particular, information about the level of RRM1 expression can be used in selecting specific methods of treatment.

RRM2 (also known as ribonucleotide reductase regulatory subunit M2) along with RRM1 constitute ribonucleoside-diphosphate reductase, an enzyme essential for the production of deoxyribonucleotides prior to DNA synthesis in S phase of dividing cells. In the fluoropyrimidine pathway, CDA catalyzes the reaction FUDP → FdUDP which promotes the processing of 5-FU in the cell to ultimately inhibit DNA synthesis. Thus high expression levels of ribonucleoside-diphosphate reductase in a tumor cell promotes higher sensitivity to treatment with 5-FU whereas lower to no expression helps confer resistance of tumor cells to treatment with 5-FU. Thus the presence and quantitative levels of RRM2 will indicate if the tumor cells may be sensitive or resistance to treatment with 5-FU. Accordingly, an assay that can quantitate the RRM2 protein in tumor cells is useful as part of a cancer therapy regimen and, in particular, information about the level of RRM2 expression can be used in selecting specific methods of treatment.

The methods below provide quantitative proteomics-based assays that quantify each of the measured proteins in formalin fixed tissues from cancer patients. In preferred embodiments, the tissue may be obtained from a tumor, such as a primary tumor or a secondary tumor. Data from the assays can be used to as part of improved methods of cancer therapy, for example.

The SRM/MRM assays can be used to measure relative or absolute quantitative levels of the specific peptides from each of the measured proteins and therefore provide a means of measuring by mass spectrometry the amount of each of the proteins in a given protein preparation obtained from a biological sample. More specifically, the SRM/MRM assay can measure these peptides directly in complex protein lysate samples prepared from cells procured from patient tissue samples, such as formalin-fixed cancer patient tissue. Methods of preparing protein samples from formalin-fixed tissue are described in U.S. Patent No. 7,473,532, the contents of which are hereby incorporated by references in their entirety. The methods described in U.S. Patent No. 7,473,532 may conveniently be carried out using Liquid Tissue® reagents and protocol available from Expression Pathology, Inc. (Rockville, MD).

The most widely and advantageously available form of tissue from cancer patients is formalin fixed, paraffin embedded tissue. Formaldehyde/formalin fixation of surgically removed tissue is by far the most common method of preserving cancer tissue samples worldwide and is the accepted convention for standard pathology practice. The formalin fixed, paraffin embedded tissue may preferably be obtained from a tumor, such as a primary tumor or a secondary tumor. Aqueous solutions of formaldehyde are referred to as formalin. "100%" formalin consists of a saturated solution of formaldehyde (about 40% by volume or 37% by mass) in water, with a small amount of stabilizer, usually methanol, to limit oxidation and degree of polymerization. The most common way in which tissue is preserved is to soak whole tissue for extended periods of time (8 hours to 48 hours) in aqueous formaldehyde, commonly termed 10% neutral buffered formalin, followed by embedding the fixed whole tissue in paraffin wax for long term storage at room temperature. Thus molecular analytical methods to analyze formalin fixed cancer tissue will be the most accepted and heavily utilized methods for analysis of cancer patient tissue.

Results from the presently-described SRM/MRM assays can be used to correlate accurate and precise quantitative levels of each of the specified proteins within the specific tissue samples (e.g., cancer tissue sample) of the patient or subject from whom the tissue (biological sample) was collected and preserved. This not only provides diagnostic information about the cancer, but also permits a physician or other medical professional to determine appropriate therapy for the patient. Such an assay that provides diagnostically and therapeutically important information about levels of protein expression in a diseased tissue or other patient sample is termed a companion diagnostic assay. For example, such an assay can be designed to diagnose the stage or degree of a cancer and determine a therapeutic agent to which a patient is most likely to respond.

Relative quantitative levels of each of the proteins are determined by the SRM/MRM methodology for example by comparing SRM/MRM signature peak areas (e.g., signature peak area or integrated fragment ion intensity) of an individual fragment peptide derived from a protein in different samples. Alternatively, it is possible to compare multiple SRM/MRM signature peak areas for multiple signature peptides, where each peptide has its own specific SRM/MRM signature peak, to determine the relative protein content in one biological sample with the content of the same protein(s) in one or more additional or different biological samples. In this way, the amount of a particular peptide, or peptides, from the subject protein(s), and therefore the amount of the designated protein(s), is determined relative to the same peptide, or peptides, across 2 or more biological samples under the same experimental conditions. In addition, relative quantitation can be determined for a given peptide, or peptides, from a given protein within a single sample by comparing the signature peak area for that peptide by SRM/MRM methodology to the signature peak area for another and different peptide, or peptides, from a different protein, or proteins, within the same protein preparation from the biological sample. In this way, the amount of a particular peptide from a designated protein, and therefore the amount of that protein, is determined relative one to another within the same sample. These approaches generate quantitation of an individual peptide, or peptides, from a designated protein to the amount of another peptide, or peptides, between samples and within samples wherein the amounts as determined by signature peak area are relative one to another, regardless of the absolute weight to volume or weight to weight amounts of the selected peptide in the protein preparation from the biological sample. Relative quantitative data about individual signature peak areas between different samples are normalized to the amount of protein analyzed per sample. Relative quantitation can be performed across many peptides from multiple proteins and one or more of the designated proteins simultaneously in a single sample and/or across many samples to gain insight into relative protein amounts, such as one peptide/protein with respect to other peptides/proteins.

Absolute quantitative levels of the designated protein are determined by, for example, the SRM/MRM methodology where the SRM/MRM signature peak area of an individual peptide from the designated protein in a biological sample is compared to the SRM/MRM signature peak area of a spiked internal standard. In one embodiment, the internal standard is a synthetic version of the same exact peptide derived from the designated protein that contains one or more amino acid residues labeled with one or more heavy isotopes. Such isotope labeled internal standards are synthesized so that when analyzed by mass spectrometry a standard generates a predictable and consistent SRM/MRM signature peak that is different and distinct from the native peptide signature peak and which can be used as a comparator peak. Thus when the internal standard is spiked into a protein preparation from a biological sample in known amounts and analyzed by mass spectrometry, the SRM/MRM signature peak area of the native peptide is compared to the SRM/MRM signature peak area of the internal standard peptide, and this numerical comparison indicates either the absolute molarity and/or absolute weight of the native peptide present in the original protein preparation from the biological sample. Absolute quantitative data for fragment peptides are displayed according to the amount of protein analyzed per sample. Absolute quantitation can be performed across many peptides, and thus proteins, simultaneously in a single sample and/or across many samples to gain insight into absolute protein amounts in individual bio logical samples and in entire cohorts of individual samples.

The SRM/MRM assay method can be used to aid diagnosis of the stage of cancer, for example, directly in patient-derived tissue, such as formalin fixed tissue, and to aid in determining which therapeutic agent would be most advantageous for use in treating that patient. Cancer tissue that is removed from a patient either through surgery, such as for therapeutic removal of partial or entire tumors, or through biopsy procedures conducted to determine the presence or absence of suspected disease, is analyzed to determine whether or not a specific protein, or proteins, and which forms of proteins, are present in that patient tissue. Thus, in a preferred embodiment, the tissue is obtained from a tumor, such as a primary tumor or a secondary tumor. Moreover, the expression level of a protein, or multiple proteins, can be determined and compared to a "normal" or reference level found in healthy tissue. Normal or reference levels of proteins found in healthy tissue may be derived from, for example, the relevant tissues of one or more individuals that do not have cancer. Alternatively, normal or reference levels may be obtained for individuals with cancer by analysis of relevant tissues not affected by the cancer. Assays of protein levels from one, some, or all of the designated proteins can also be used to diagnose the stage of cancer in a patient or subject diagnosed with cancer by employing the protein levels. The level of an individual peptide derived from a designated protein is defined as the molar amount of the peptide determined by the SRM/MRM assay per total amount of protein lysate analyzed. Information regarding a designated protein or proteins can thus be used to aid in determining the stage or grade of a cancer by correlating the level of the protein(s) (or fragment peptides from the proteins) with levels observed in normal tissues. Once the quantitative amount of one or more of the designated proteins has been determined in the cancer cells, that information can be matched to a list of therapeutic agents (chemical and biological) developed to specifically treat cancer tissue that is characterized by, for example, abnormal expression of the protein or protein(s) that were assayed. Matching information from a protein assay to a list of therapeutic agents that specifically targets, for example, the designated protein or cells/tissue expressing the protein, defines what has been termed a personalized medicine approach to treating disease. The assay methods described herein form the foundation of a personalized medicine approach by using analysis of proteins from the patient's own tissue as a source for diagnostic and treatment decisions.

In principle, any predicted peptide derived from a designated protein, prepared for example by digesting with a protease of known specificity (e.g. trypsin), can be used as a surrogate reporter to determine the abundance of a designated protein in a sample using a mass spectrometry-based SRM/MRM assay. Similarly, any predicted peptide sequence containing an amino acid residue at a site that is known to be potentially modified in the designated protein also might potentially be used to assay the extent of modification of the designated protein in a sample.

Suitable fragment peptides derived from a designated protein may be generated by a variety of means including by the use of the Liquid Tissue® protocol provided in US Patent 7,473,532. The Liquid Tissue® protocol and reagents are capable of producing peptide samples suitable for mass spectroscopic analysis from formalin fixed paraffin embedded tissue by proteolytic digestion of the proteins in the tissue/biological sample. In the Liquid Tissue® protocol the tissue/biological is heated in a buffer for an extended period of time (e.g., from about 80° C to about 100° C for a period of time from about 10 minutes to about 4 hours) to reverse or release protein cross-linking. The buffer employed is a neutral buffer, (e.g., a Tris-based buffer, or a buffer containing a detergent). Following heat treatment the tissue/biological sample is treated with one or more proteases, including but not limited to trypsin, chymotrypsin, pepsin, and endoproteinase Lys-C for a time sufficient to disrupt the tissue and cellular structure of said biological sample. The result of the heating and proteolysis is a liquid, soluble, dilutable biomolecule lysate.

Surprisingly, it was found that many potential peptide sequences from the proteins listed above are unsuitable or ineffective for use in mass spectrometry-based SRM/MRM assays for reasons that are not immediately evident. As it was not possible to predict the most suitable peptides for an individual SRM/MRM assay, it was necessary to experimentally identify modified and unmodified peptides in actual Liquid Tissue® lysates to develop a reliable and accurate SRM/MRM assay for each designated protein. While not wishing to be bound by any theory, it is believed that some peptides might, for example, be difficult to detect by mass spectrometry because they do not ionize well or produce fragments distinct from other proteins. Peptides may also fail to resolve well in separation (e.g., liquid chromatography), or may adhere to glass or plastic ware.

The peptides found in Table 1 were derived from the respective designated proteins by protease digestion of all the proteins within a complex Liquid Tissue® lysate prepared from cells procured from formalin fixed cancer tissue. Unless noted otherwise, in each instance the protease was trypsin. The Liquid Tissue® lysate was then analyzed by mass spectrometry to determine those peptides derived from a designated protein that are detected and analyzed by mass spectrometry. Identification of a specific preferred subset of peptides for mass spectrometric analysis is based on; 1) experimental determination of which peptide or peptides from a protein ionize in mass spectrometry analyses of Liquid Tissue lysates, and 2) the ability of the peptide to survive the protocol and experimental conditions used in preparing a Liquid Tissue® lysate. This latter property extends not only to the amino acid sequence of the peptide but also to the ability of a modified amino acid residue within a peptide to survive in modified form during the sample preparation.

Protein lysates from cells procured directly from formalin (formaldehyde) fixed tissue were prepared using the Liquid Tissue® reagents and protocol that entails collecting cells into a sample tube via tissue microdissection followed by heating the cells in the Liquid Tissue® buffer for an extended period of time. Once the formalin-induced cross-linking has been negatively affected, the tissue/cells are then digested to completion in a predictable manner using a protease, as for example including but not limited to the protease trypsin. Each protein lysate is reduced to a collection of peptides by digestion of intact polypeptides with the protease. Each Liquid Tissue® lysate was analyzed (e.g., by ion trap mass spectrometry) to perform multiple global proteomic surveys of the peptides where the data was presented as identification of as many peptides as could be identified by mass spectrometry from all cellular proteins present in each protein lysate. An ion trap mass spectrometer or another form of a mass spectrometer that is capable of performing global profiling for identification of as many peptides as possible from a single complex protein/peptide lysate is typically employed.

Although an SRM/MRM assay can be developed and performed on any type of mass spectrometer, including a MALDI, ion trap, or triple quadrupole, advantageously a triple quadrupole instrument platform is used. This type of a mass spectrometer is able to analyze a single isolated target peptide within a very complex protein lysate that may consist of hundreds of thousands to millions of individual peptides from all the proteins contained within a cell. The described method was used to: 1) identify candidate peptides from each designated protein that can be used for a mass spectrometry-based SRM/MRM assay for the designated protein, 2) develop an individual SRM/MRM assay, or assays, for target peptides from the designated protein in order to correlate and 3) apply quantitative assays to cancer diagnosis and/or choice of optimal therapy.

### EXAMPLES

The tryptic peptides identified as useful in the determination of absolute or relative amounts of the designated proteins are listed in Table 1. Each of these peptides was detected by mass spectrometry in Liquid Tissue® lysates prepared from formalin fixed, paraffin embedded tissue. Thus, each peptide can be used in a quantitative SRM/MRM assay for a designated protein in human biological samples, including directly in formalin fixed patient tissue.

**Table 1**

| **SEQ ID NO** | **Protein** | **Peptide Sequence** |
|---|---|---|
| SEQ ID NO 1 | TYMS | EEGDLGPVYGFQWR |
| SEQ ID NO 2 | TYMS | DFLDSLGFSTR |
| SEQ ID NO 3 | TYMP | DGPALSGPQSR |
| SEQ ID NO 4 | TYMP | VAAALDDGSALGR |
| SEQ ID NO 5 | UCK1 | VLTAEQK |
| SEQ ID NO 6 | UCK1 | WILSQDR |
| SEQ ID NO 7 | UCK2 | LFVDTDADTR |
| SEQ ID NO 8 | UCK2 | IVQLLGQNEVDYR |
| SEQ ID NO 9 | UPP1 | LEGDQISSPR |
| SEQ ID NO 10 | UPP1 | AEFEQIVLGK |
| SEQ ID NO 11 | OPRT | ELSFGAR |
| SEQ ID NO 12 | OPRT | VTDAIVLLDR |
| SEQ ID NO 13 | dCK | STFVNILK |
| SEQ ID NO 14 | dCK | YESLVEK |
| SEQ ID NO 15 | TK1 | FQIAQYK |
| SEQ ID NO 16 | TK1 | EVEVIGGADK |
| SEQ ID NO 17 | DPYD | DFLPLVAK |
| SEQ ID NO 18 | DPYD | SFITSIANK |
| SEQ ID NO 19 | CDA | AVSEGYK |
| SEQ ID NO 20 | CDA | GCNIENACYPLGICAER |
| SEQ ID NO 21 | RRM1 | TVWEISQK |
| SEQ ID NO 22 | RRM1 | LNSAIIYDR |
| SEQ ID NO 23 | RRM2 | AAAPGVEDEPLLR |
| SEQ ID NO 24 | RRM2 | EIIINAVR |

The tryptic peptides listed in Table 1 typically were detected from multiple Liquid Tissue®lysates of multiple different formalin fixed tissues of different human organs including, for example, prostate, colon, and breast.

### Assay Method

### 1. Identification of SRM/MRM candidate fragment peptides for a protein

a. Prepare a Liquid Tissue® protein lysate from a formalin fixed biological sample using a protease or proteases, (that may or may not include trypsin), to digest proteins.
b. Analyze all protein fragments in the Liquid Tissue® lysate on an ion trap tandem mass spectrometer and identify all fragment peptides from a designated protein, where individual fragment peptides do not contain any peptide modifications such as phosphorylations or glycosylations.
c. Analyze all protein fragments in the Liquid Tissue® lysate on an ion trap tandem mass spectrometer and identify all fragment peptides from the protein that carry peptide modifications such as for example phosphorylated or glycosylated residues.
d. All peptides generated by a specific digestion method from an entire, full length protein can potentially be measured, but preferred peptides used for development of the SRM/MRM assay are those that are identified by mass spectrometry directly in a complex Liquid Tissue® protein lysate prepared from a formalin fixed biological sample.

### 2. Mass Spectrometry Assay for Fragment Peptides from a Designated Protein

a. An SRM/MRM assay on a triple quadrupole mass spectrometer for individual fragment peptides identified in a Liquid Tissue® lysate is applied to peptides from the protein.
   i. Determine optimal retention time for a fragment peptide for optimal chromatography conditions including but not limited to gel electrophoresis, liquid chromatography, capillary electrophoresis, nano-reversed phase liquid chromatography, high performance liquid chromatography, or reverse phase high performance liquid chromatography.
   ii. Determine the mono isotopic mass of the peptide, the precursor charge state for each peptide, the precursor m/z value for each peptide, the m/z transition ions for each peptide, and the ion type of each transition ion for each fragment peptide in order to develop an SRM/MRM assay for each peptide.
   iii. An SRM/MRM assay can then be conducted using the information from (i) and (ii) on a triple quadrupole mass spectrometer where each peptide has a characteristic and unique SRM/MRM signature peak that precisely defines the unique SRM/MRM assay as performed on a triple quadrupole mass spectrometer.
b. Perform SRM/MRM analysis so that the amount of the fragment peptide of the protein that is detected, as a function of the unique SRM/MRM signature peak area from an SRM/MRM mass spectrometry analysis, can indicate both the relative and absolute amount of the protein in a particular protein lysate.
   i. Relative quantitation may be achieved by:
      1. Determining increased or decreased presence of the protein by comparing the SRM/MRM signature peak area from a given fragment peptide detected in a Liquid Tissue® lysate from one formalin fixed biological sample to the same SRM/MRM signature peak area of the same fragment peptide in at least a second, third, fourth or more Liquid Tissue® lysates from least a second, third, fourth or more formalin fixed biological samples.
      2. Determining increased or decreased presence of the protein by comparing the SRM/MRM signature peak area from a given fragment peptide detected in a Liquid Tissue® lysate from one formalin fixed biological sample to SRM/MRM signature peak areas developed from fragment peptides from other proteins, in other samples derived from different and separate biological sources, where the SRM/MRM signature peak area comparison between the 2 samples for a peptide fragment are normalized to amount of protein analyzed in each sample.
      3. Determining increased or decreased presence of the protein by comparing the SRM/MRM signature peak area for a given fragment peptide to the SRM/MRM signature peak areas from other fragment peptides derived from different proteins within the same Liquid Tissue® lysate from the formalin fixed biological sample in order to normalize changing levels of a protein to levels of other proteins that do not change their levels of expression under various cellular conditions.
      4. These assays can be applied to both unmodified fragment peptides and for modified fragment peptides of the protein, where the modifications include but are not limited to phosphorylation and/or glycosylation, and where the relative levels of modified peptides are determined in the same manner as determining relative amounts of unmodified peptides.
   ii. Absolute quantitation of a given peptide may be achieved by comparing the SRM/MRM signature peak area for a given fragment peptide from the designated protein in an individual biological sample to the SRM/MRM signature peak area of an internal fragment peptide standard spiked into the protein lysate from the biological sample.
      1. The internal standard is a labeled synthetic version of the fragment peptide from the designated protein that is being interrogated. This standard is spiked into a sample in known amounts, and the SRM/MRM signature peak area can be determined for both the internal fragment peptide standard and the native fragment peptide in the biological sample separately, followed by comparison of both peak areas.
      2. This can be applied to unmodified fragment peptides and modified fragment peptides, where the modifications include but are not limited to phosphorylation and/or glycosylation, and where the absolute levels of modified peptides can be determined in the same manner as determining absolute levels of unmodified peptides.

### 3. Apply Fragment Peptide Quantitation to Cancer Diagnosis and Treatment

a. Perform relative and/or absolute quantitation of fragment peptide levels of the designated protein and demonstrate that the previously-determined association, as well understood in the field of cancer, of expression of the designated protein to the stage/grade/status of cancer in patient tumor tissue is confirmed.
b. Perform relative and/or absolute quantitation of fragment peptide levels of the designated protein and demonstrate correlation with clinical outcomes from different treatment strategies, wherein this correlation has already been demonstrated in the field or can be demonstrated in the future through correlation studies across cohorts of patients and tissue from those patients. Once either previously established correlations or correlations derived in the future are confirmed by this assay then the assay method can be used to determine optimal treatment strategy.

Specific and unique characteristics about specific fragment peptides from each designated protein were developed by analysis of all fragment peptides on both an ion trap and triple quadrupole mass spectrometers. That information must be determined experimentally for each and every candidate SRM/MRM peptide directly in Liquid Tissue lysates from formalin fixed samples/tissue; because, interestingly, not all peptides from any designated protein can be detected in such lysates using SRM/MRM as described herein, indicating that fragment peptides not detected cannot be considered candidate peptides for developing an SRM/MRM assay for use in quantitating peptides/proteins directly in Liquid Tissue lysates from formalin fixed samples/tissue.

A particular SRM/MRM assay for a specific fragment peptide is performed on a triple quadrupole mass spectrometer. An experimental sample analyzed by a particular protein SRM/MRM assay is for example a Liquid Tissue protein lysate prepared from a tissue that had been formalin fixed and paraffin embedded. Data from such as assay indicates the presence of the unique SRM/MRM signature peak for this fragment peptide in the formalin fixed sample.

Specific transition ion characteristics for a given peptide are used to quantitatively measure that given fragment peptide in formalin fixed biological samples. These data indicate absolute amounts of this fragment peptide as a function of the molar amount of the peptide per microgram of protein lysate analyzed. Assessment of corresponding protein levels in tissues based on analysis of formalin fixed patient-derived tissue can provide diagnostic, prognostic, and therapeutically-relevant information about each particular patient. In one embodiment, this disclosure describes a method for measuring the level of each of the proteins listed in Table 1 in a biological sample, comprising detecting and/or quantifying the amount of one or more modified or unmodified fragment peptides in a protein digest prepared from said biological sample using mass spectrometry; and calculating the level of modified or unmodified protein in said sample; and wherein said level is a relative level or an absolute level. In a related embodiment, quantifying one or more fragment peptides comprises determining the amount of the each of the fragment peptides in a biological sample by comparison to an added internal standard peptide of known amount, wherein each of the fragment peptides in the biological sample is compared to an internal standard peptide having the same amino acid sequence. In some embodiments the internal standard is an isotopically labeled internal standard peptide comprises one or more heavy stable isotopes selected from ¹⁸O, ¹⁷O, ³⁴S , ¹⁵N, ¹³C, ²H or combinations thereof.

The method for measuring the level of a designated protein a biological sample described herein (or fragment peptides as surrogates thereof) may be used as a diagnostic indicator of cancer in a patient or subject, and an indicator of the most optimal therapy for that patient or subject. In one embodiment, the results from measurements of the level of a designated protein may be employed to determine the diagnostic stage/grade/status of a cancer by correlating (e.g., comparing) the level of the protein found in a tissue with the level of that protein found in normal and/or cancerous or precancerous tissues. In another embodiment, the results from measurements of the level of a designated protein may be employed to determine the most optimal therapeutic agent (drug) with which to treat the patient by correlating (e.g., comparing) the level of the protein found in patient tumor cells whereby the protein is a target of a specific cancer therapeutic agent (drug).

Because both nucleic acids and protein can be analyzed from the same Liquid Tissue® biomolecular preparation it is possible to generate additional information about disease diagnosis and drug treatment decisions from the nucleic acids in same sample upon which proteins were analyzed. For example, if a designated protein is expressed by certain cells at increased levels, when assayed by SRM/MRM the data can provide information about the state of the cells and their potential for uncontrolled growth, potential drug resistance/sensitivity, and the development of other cancers. At the same time, information about the status of the corresponding genes and/or the nucleic acids and proteins they encode (e.g., mRNA molecules and their expression levels or splice variations) can be obtained from nucleic acids present in the same Liquid Tissue® biomolecular preparation that can be assessed simultaneously to the SRM/MRM analysis of the designated protein. Any gene and/or nucleic acid that does not code for the designated protein and which is present in the same biomolecular preparation can be assessed simultaneously to the SRM/MRM analysis of the designated protein. In one embodiment, information about the designated protein and/or one, two, three, four or more additional proteins may be assessed by examining the nucleic acids encoding those proteins. Those nucleic acids can be examined, for example, by one or more, two or more, or three or more of: sequencing methods, polymerase chain reaction methods, restriction fragment polymorphism analysis, identification of deletions, insertions, and/or determinations of the presence of mutations, including but not limited to, single base pair polymorphisms, transitions, transversions, or combinations thereof.

## Claims

1. A method for measuring a level of protein in a biological sample of formalin fixed tissue, comprising
detecting and/or quantifying an amount of one or more modified or unmodified fragment peptides derived from the protein in a protein digest prepared from said biological sample of formalin fixed tissue using mass spectrometry; and
calculating the level of said protein in said biological sample of formalin fixed tissue;
wherein said protein is selected from the group consisting of TYMS, TYMP, UCK1, UCK2, UPP1, OPRT, dCK, TK1, DPYD, CDA, RRM1 and RRM2.

2. The method of claim 1, further comprising the step of fractionating said protein digest prior to detecting and/or quantifying the amount of said one or more modified or unmodified fragment peptides.

3. The method of claim 2, wherein said fractionating step is selected from the group consisting of liquid chromatography, nanoreversed phase liquid chromatography, high performance liquid chromatography, or reverse phase high performance liquid chromatography.

4. The method of any one of claims 1-3, wherein said protein digest comprises a protease digest, preferably a trypsin digest.

5. The method of any one of claims 1-5, wherein said mass spectrometry comprises tandem mass spectrometry, ion trap mass spectrometry, triple quadrupole mass spectrometry, ion trap/quadrupole hybrid mass spectrometry, MALDI-TOF mass spectrometry, MALDI mass spectrometry, and/or time of flight mass spectrometry.

6. The method of claim 5, wherein the mode of mass spectrometry used is Selected Reaction Monitoring (SRM), Multiple Reaction Monitoring (MRM), intelligent Selected Reaction Monitoring (iSRM), and/or multiple Selected Reaction Monitoring (mSRM).

7. The method of any one of claims 1 to 6, wherein
said protein is TYMS and said fragment peptides are the peptides of SEQ ID NO:1 and/or SEQ ID NO:2; or
said protein is TYMP and said fragment peptides are the peptides of SEQ ID NO:3 and/or SEQ ID NO:4; or
said protein is UCK1 and said fragment peptides are the peptides of SEQ ID NO:5 and/or SEQ ID NO:6; or
said protein is UCK2 and said fragment peptides are the peptides of SEQ ID NO:7 and/or SEQ ID NO:8; or
said protein is UPP1 and said fragment peptides are the peptides of SEQ ID NO:9 and/or SEQ ID NO:10; or
said protein is OPRT and said fragment peptides are the peptides of SEQ ID NO:11 and/or SEQ ID NO:12; or
said protein is dCK and said fragment peptides are the peptides of SEQ ID NO:13 and/or SEQ ID NO:14; or
said protein is TK1 and said fragment peptides are the peptides of SEQ ID NO:15 and/or SEQ ID NO:16; or
said protein is DPYD and said fragment peptides are the peptides of SEQ ID NO:17 and/or SEQ ID NO:18; or
said protein is CDA and said fragment peptides are the peptides of SEQ ID NO:19 and/or SEQ ID NO:20; or
said protein is RRM1 and said fragment peptides are the peptides of SEQ ID NO:21 and/or SEQ ID NO:22; or
said protein is RRM2 and said fragment peptides are the peptides of SEQ ID NO:23 and/or SEQ ID NO:24.

8. The method of any preceding claim, wherein said tissue is paraffin embedded tissue, preferably obtained from a tumor such as a primary tumor or a secondary tumor.

9. The method of any one of claims 1 to 8, wherein at least one fragment peptide is quantified.

10. The method of claim 9, wherein quantifying said fragment peptide comprises comparing an amount of said fragment peptide in one biological sample to the amount of the same fragment peptide in a different and separate biological sample.

11. The method of claim 9, wherein quantifying said fragment peptide comprises determining the amount of said fragment peptide in a biological sample by comparison to an added internal standard peptide of known amount having the same amino acid sequence.

12. The method of claim 11, wherein the internal standard peptide is an isotopically labeled peptide selected from the group consisting of ¹⁸O, ¹⁷O, ³⁴S, ¹⁵N, ¹³C, and ²H or a combination thereof.

13. The method of any one of claims 1 to 12, wherein detecting and/or quantifying the amount of at least one fragment peptide in the protein digest indicates the presence of the corresponding protein and an association with the diagnostic stage/grade/status of cancer in the subject.

14. The method of any one of claims 1 to 13, further comprising correlating the results of said detecting and/or quantifying the amount of said at least one fragment peptide, or the level of the corresponding protein to informing an optimal cancer treatment therapy for the subject.

15. The method of claim 14, wherein correlating the results of said detecting and/or quantifying the amount of said at least one fragment peptide or the level of said corresponding protein to informing an optimal cancer treatment therapy for the subject is combined with detecting and/or quantifying the amount of other proteins or peptides from other proteins in a multiplex format to provide additional information about an optimal cancer treatment therapy for the subject.


SEQUENCE LISTING

<110>  NantOmics, LLC

<120>  IDENTIFICATION OF CANCER PATIENTS SENSITIVE OR RESISTANT TO
       5-FLUOROURACIL (5-FU)

<130>  N416647EP

<150>  62/672671
<151>  2018-05-17

<160>  24

<170>  PatentIn version 3.5

<210>  1
<211>  14
<212>  PRT
<213>  Homo sapiens

<400>  1

Glu Glu Gly Asp Leu Gly Pro Val Tyr Gly Phe Gln Trp Arg
1               5                   10


<210>  2
<211>  11
<212>  PRT
<213>  Homo sapiens

<400>  2

Asp Phe Leu Asp Ser Leu Gly Phe Ser Thr Arg
1               5                   10


<210>  3
<211>  11
<212>  PRT
<213>  Homo sapiens

<400>  3

Asp Gly Pro Ala Leu Ser Gly Pro Gln Ser Arg
1               5                   10


<210>  4
<211>  13
<212>  PRT
<213>  Homo sapiens

<400>  4

Val Ala Ala Ala Leu Asp Asp Gly Ser Ala Leu Gly Arg
1               5                   10


<210>  5
<211>  7
<212>  PRT

<213>  Homo sapiens

<400>  5

Val Leu Thr Ala Glu Gln Lys
1               5


<210>  6
<211>  8
<212>  PRT
<213>  Homo sapiens

<400>  6

Val Val Ile Leu Ser Gln Asp Arg
1               5


<210>  7
<211>  10
<212>  PRT
<213>  Homo sapiens

<400>  7

Leu Phe Val Asp Thr Asp Ala Asp Thr Arg
1               5                   10


<210>  8
<211>  13
<212>  PRT
<213>  Homo sapiens

<400>  8

Ile Val Gln Leu Leu Gly Gln Asn Glu Val Asp Tyr Arg
1               5                   10


<210>  9
<211>  10
<212>  PRT
<213>  Homo sapiens

<400>  9

Leu Glu Gly Asp Gln Ile Ser Ser Pro Arg
1               5                   10


<210>  10
<211>  10
<212>  PRT
<213>  Homo sapiens

<400>  10

Ala Glu Phe Glu Gln Ile Val Leu Gly Lys
1               5                   10

<210>  11
<211>  7
<212>  PRT
<213>  Homo sapiens

<400>  11

Glu Leu Ser Phe Gly Ala Arg
1               5


<210>  12
<211>  10
<212>  PRT
<213>  Homo sapiens

<400>  12

Val Thr Asp Ala Ile Val Leu Leu Asp Arg
1               5                   10


<210>  13
<211>  8
<212>  PRT
<213>  Homo sapiens

<400>  13

Ser Thr Phe Val Asn Ile Leu Lys
1               5


<210>  14
<211>  7
<212>  PRT
<213>  Homo sapiens

<400>  14

Tyr Glu Ser Leu Val Glu Lys
1               5


<210>  15
<211>  7
<212>  PRT
<213>  Homo sapiens

<400>  15

Phe Gln Ile Ala Gln Tyr Lys
1               5


<210>  16
<211>  10
<212>  PRT
<213>  Homo sapiens

<400>  16

Glu Val Glu Val Ile Gly Gly Ala Asp Lys

1               5                   10


<210>  17
<211>  8
<212>  PRT
<213>  Homo sapiens

<400>  17

Asp Phe Leu Pro Leu Val Ala Lys
1               5


<210>  18
<211>  9
<212>  PRT
<213>  Homo sapiens

<400>  18

Ser Phe Ile Thr Ser Ile Ala Asn Lys
1               5


<210>  19
<211>  7
<212>  PRT
<213>  Homo sapiens

<400>  19

Ala Val Ser Glu Gly Tyr Lys
1               5


<210>  20
<211>  17
<212>  PRT
<213>  Homo sapiens

<400>  20

Gly Cys Asn Ile Glu Asn Ala Cys Tyr Pro Leu Gly Ile Cys Ala Glu
1               5                   10                  15


Arg



<210>  21
<211>  8
<212>  PRT
<213>  Homo sapiens

<400>  21

Thr Val Trp Glu Ile Ser Gln Lys
1               5


<210>  22

<211>  9
<212>  PRT
<213>  Homo sapiens

<400>  22

Leu Asn Ser Ala Ile Ile Tyr Asp Arg
1               5


<210>  23
<211>  13
<212>  PRT
<213>  Homo sapiens

<400>  23

Ala Ala Ala Pro Gly Val Glu Asp Glu Pro Leu Leu Arg
1               5                   10


<210>  24
<211>  8
<212>  PRT
<213>  Homo sapiens

<400>  24

Glu Ile Ile Ile Asn Ala Val Arg
1               5